Europäisches Patentamt

(19) European Patent Office

Office européen des brevets

(11) Publication number: **0 251 971**
**A2**

(12) # EUROPEAN PATENT APPLICATION

(21) Application number: **87500034.1**

(22) Date of filing: **16.06.87**

(51) Int. Cl.⁴: **A 61 B 17/39**

(30) Priority: **25.06.86 ES 556589**

(43) Date of publication of application:
**07.01.88 Bulletin 88/01**

(84) Designated Contracting States:
**AT BE CH DE FR GB GR IT LI NL SE**

(71) Applicant: **FUCHELMAN, s.a.**
**Artilleria de Montana, 5**
**E-07014 Palma de Mallorca (ES)**

(72) Inventor: **Fuchelman Gross, Julio Ricardo**
**Artilleria de Montana 5**
**E-07014 Palma de Mallorca (ES)**

(74) Representative: **Urteaga Simarro, José Antonio**
**Gran Via, 86(6-5-5)**
**E-28013 Madrid (ES)**

(54) **Contour type electrosurgical dispersive electrode.**

(57) Contour type electrosurgical dispersive electrode3charac-
terized by the fact of having a contact surface with the skin in
which its perimeter electrically and/or geometrically bounds a
region of the surface of the body which contains the surgical
field, formed by one or more electrically conductive zones
linked together in a conductive and/or capacitive way which
when in a conductive, capacitive or conductive and capacitive
contact with the skin permits the return path for the RFC
generated by the ESU.

FIG.2

**Description**

## CONTOUR TYPE ELECTROSURGICAL DISPERSIVE ELECTRODE

The invention referred to herein is a new concept of the part of an electrosurgical unit which is known as dispersive, neutral or feedback electrode.

The new development originates from a theoretical and experimental analysis that shows that the efficient electrode area for the current collection is found in the peripheral zone of the electrode which is in contact with the patient's skin.

An electrosurgical unit comprises three main parts: one radiofrecuency generator -1-, an active electrode -2- and a neutral or dispersive electrode -3-. Until now the dispersive electrodes have been designed with a criterion according to safety standards, that is to say 1 square centimeter per 1,5 Watts of radiofrecuency power. This criterion fails because until now manufacturers have not taken into account that the RFC isn't evenly distributed over the surface of the dispersive plate -5-but almost all ofthe current is collected in 20% of the electrode area, which is found in the peripheral zone of the electrode in contact with the patient's skin.

In order to have a correct understanding of the objective of this invention, we hereby enclose a sheet of drawings, in which, by way of example, each and everyone of the parts that form the invention are outlined.

In the above mentioned sheet of drawings, the following are listed:

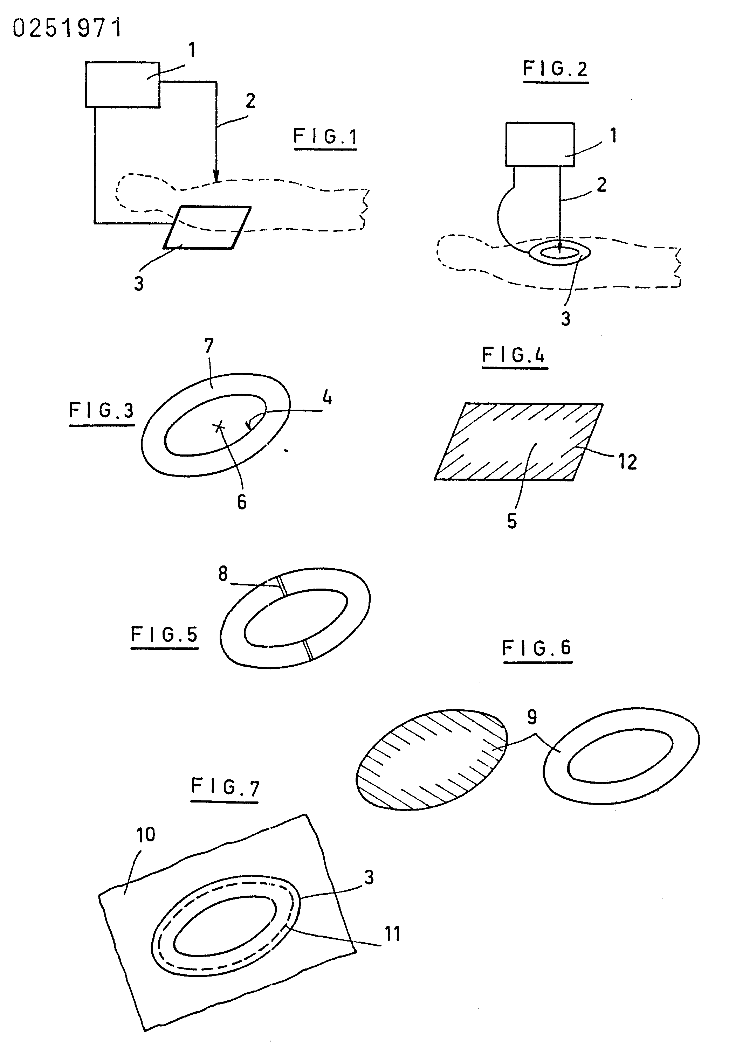

FIGURE 1. - Shows the normal way in which a conventional electrosurgical dispersive electrode is used.

FIGURE 2. - Shows an example of the normal way in which the contour type electrosurgical dispersive electrode is used.

FIGURE 3. - Shows one of the many geometrical shapes that this invention can adopt.

FIGURE 4. - Shows a conventional dispersive electrode in which its functional area is lined.

FIGURE 5. - Shows the objective of this patent which is made up of one or more conductive regions.

FIGURE 6. - Is a comparative example of a conventional electrode and the one proposed in the invention herein.

FIGURE 7. - Is one example of the use of the plate fixed with and by an adhesive membrane.

In the above mentioned figures the following main parts are referred to in addition to the ones already mentioned.

The new contour type electrode -3- which is the objective of this patent, is hollow in the centre -4- differs from the conventional type constituted by a simple connected surface (without holes) -5- and in addition to providing a return for the RFC, bounds the area through which the RFC flows to a region which is practically equal to the surface of the patient's skin which, containing the surgical incision line made by the active electrode of the ESU -6- is bounded at the same time by the perimeter of the contour type dispersive electrode.7

Taking into account the low penetration depth of the RFC in an electrically conductive material, this type of dispersive plate can be manufactured to a minimal thickness, thereby making it light, flexible and economical.

Because of its flexibility and light weight it can be fixed to the surface of the body by means of adhesives methods

This plate is formed by one or more conductive zones-8-electrically linked together, either in a conductive or capacitive way, either on the plate itself or in the return lead to the ESU.

Contact with the skin can be either conductive, capacitive or a combination of both.

This new development of collecting the radiofrequency current by means of contour type dispersive electrode and where a 100% of the skin contact area is active in the RFC collection gives rise to the following advantages:

A. - Minimizes the RFC burns, as all the area which is taken into account when calculating the density of the current, is active.9

B.- Minimizes the probability of interaction between the RFC of the ESU and other equipment which is also connected to the patient: e.g. anaethesia cardiac monitors, etc.

C.- Minimizes the probability of interaction between the biological processes of the body and the RFC, as the latter can only flow through a region which is practically equal to the surface of the patient skin which, containing the surgical incision, is bounded at the same time by the perimeter of the contour type dispersive electrode.

D.- Can be easily and quickly disconnected.

E.- It increases the safety of the surgeon and his team as the spreading of the RFC is avoided.

F.- Owing to its shape which enables it to have a maximum proximity between the point of contact of the active electrode with the skin and the dispersive electrode the impedance between these two electrodes is minimized, at the same time improving its performance and increasing the safety precautions in the work carried out.

G.- Because of its contour type shape it can be fixed to the patient's skin bounding the aseptic surgical field, and for this reason it can be considered to be 100% radiotransparent, and in spite of its maximum proximity to the surgical site it does not cause any interference with the X-rays.

H.- Because of its contour type shape,(which is economical and flexible), it can be promoted and applied as an unique product together with an adhesive membrane -10- the latter acting as an adherent for the dispersive electrode and at the same time giving protection from bacteria in the surgical zone.

I.- Because of its contour type shape it can be manufactured with opaque lines to the X-ray that give a radioscopic information about the boundary of the surgical field.

With reference to the aforementioned description, it is necessary to emphasize that the details of the performance of the proposed idea can vary, that is to say, they can undergo slight alterations which are always based on the fundamental principles of the idea of a contour type dispersive electrode.

## Claims

1. Contour type electrosurgical dispersive electrode, characterized by the fact of having a contact surface with the skin in which its perimeter electrically and/or geometrically bounds a region of the surface of the body which contains the surgical field.

2. Contour type electrosurgical dispersive electrode, as already stated in the first claim, formed by one or more electrically conductive zones linked together in a conductive and/or capacitive way (conductive and/or displacement current) which when in a conductive, capacitive or conductive and capacitive contact with the skin permits the return path for the RFC generated by the ESU.

3. Contour type electrosurgical dispersive electrode, as already stated in the first and second claims characterized by its ability to include an adhesive membrane which is used for mechanical fixing.

4. Contour type electrosurgical dispersive electrode, as already stated in the first and second claims, characterized by the ability to include X-ray opaque lines that give geometrical information of the volume beneath the surgical field.

5. Contour type electrosurgical dispersive electrode.

0251971

FIG.1

FIG.2

FIG.3

FIG.4

FIG.5

FIG.6

FIG.7